# EUROPÄISCHE PATENTANMELDUNG

(11) **EP 2 090 230 A1**
(43) Veröffentlichungstag der Anmeldung: **19.08.2009**
(21) Anmeldenummer: 09001743.5
(22) Anmeldetag: 07.02.2009
(51) Int. Cl.: A61B 17/00, A61B 17/12, A61F 2/01, A61M 25/00, A61M 25/01

(54) **Vorrichtung zum Implantieren einer Verschlussvorrichtung im Herzen**

(30) Priorität: 16.02.2008 DE 102008009525
(71) Anmelder: Peter Osypka Stiftung Stiftung des bürgerlichen Rechts, 79639 Grenzach-Wyhlen (DE)
(72) Erfinder: Osypka, Peter, 79618 Rhinfelden-Herten (DE)
(74) Vertreter: Maucher, Wolfgang

(57) **Zusammenfassung**

Eine Vorrichtung (1) dient zum Implantieren eines Verschlusses (2) in eine ungewollte Öffnung in einer Trennwand (3) in einem Herzen (4) und weist einen Katheter (5) mit einem in dessen Längsrichtung im Inneren bewegbaren Schiebeelement (7) (pusher) auf, an dessen distalem Ende der Verschluss (2) lösbar befestigt ist und in seine Gebrauchsstellung gebracht werden kann, wo das Schiebeelement (7) von dem Verschluss (2) wieder gelöst wird. Dabei ist vorgesehen, dass der mit dem Schiebeelement (7) verbundene Verschluss (2) für den Implantationsvorgang im distalen Endbereich des Katheters (5) angeordnet und von diesem Endbereich ganz oder wenigstens teilweise umschlossen ist (Fig. 1).

## Beschreibung

Die Erfindung betrifft eine Vorrichtung zum Implantieren einer Verschlussvorrichtung in einer ungewollten Öffnung der Trennwand zwischen den beiden Vorhöfen eines Herzens mit einem Katheter und mit einem in diesen Katheter in dessen Längserstreckungsrichtung bewegbaren Schiebeelement, an dessen distalem Ende der Verschluss lösbar anbringbar oder lösbar befestigt und durch die Verschiebebewegung aus dem distalen Ende des Katheters in seine Gebrauchsstellung bringbar und dann von dem Schiebelement trennbar ist.

Eine vergleichbare Vorrichtung geht aus der US 6 334 864 B1 hervor. Darin wird beschrieben, dass der in der Regel aus einem Metallgeflecht bestehende Verschluss über die gesamte Länge des Katheters in das Herz zu verschieben ist, was aufgrund der Länge eines solchen Katheters oder Katheterschlauches und der dabei zu durchlaufenden Krümmungen auch deshalb Probleme mit sich bringt, weil während der Verschiebebewegung, mit welcher der Verschluss durch den Katheter hindurchbewegt wird, das Metallgeflecht dieses Verschlusses an der Innenseite des Katheters, die häufig aus Kunststoff besteht oder damit beschichtet ist, reibt und deshalb die Gefahr besteht, dass Kunststoffpartikel abgerieben und in das Herz gebracht werden.

Es besteht deshalb die Aufgabe, eine Vorrichtung der eingangs genannten Art zu schaffen, mit welcher die Gefahr eines Abriebes vom Inneren das Katheters vermindert oder ausgeschlossen ist.

Zur Lösung dieser Aufgabe ist die eingangs definierte Vorrichtung dadurch gekennzeichnet, dass der mit dem Schiebeelement (pusher) verbundene Verschluss für, vor und bei dem Implantationsvorgang im distalen Endbereich des Katheters in dessen Innerem angeordnet und von diesem Endbereich ganz oder wenigstens teilweise umschlossen ist.

Durch diese Anordnung ergibt sich, dass für das Einführen des Verschlusses aus dem Katheter heraus an den Ort der Bestimmung nur eine relativ geringe Verschiebebewegung mit entsprechend geringer Gefahr eines Abriebes erforderlich ist. Es genügt, den Katheter mit dem in dessen distalen Endbereich befindlichen Verschluss zusammen in das Herz einzuführen und danach den Verschluss aus dem distalen Ende des Katheters in die Öffnung in die Trennwand im Inneren des Herzens auszuschieben und dadurch in Gebrauchsstellung zu bringen, ohne dabei den Verschluss über die gesamte Länge des Katheters verschieben zu müssen.

Der Katheter kann eine flexible Wendel enthalten oder durch eine solche Wendel gebildet sein. Dies ergibt den Vorteil hoher Flexibilität des Katheters, der dabei das in einem inneren Lumen des Katheters befindliche Schiebelement enthalten und dennoch gut an unterschiedliche und wechselnde Richtungen eines Blutgefäßes angepasst werden kann.

Eine ganz besonders vorteilhafte Ausgestaltung der Erfindung kann vorsehen, dass das distale Ende des Katheters durch einen dort angeordneten Aufnahmeschlauch gebildet ist, der mit dem übrigen Katheter - dessen Längserstreckungsrichtung fortsetzend - verbunden ist und den Verschluss zumindest teilweise derart in sich aufnimmt, dass das distale Ende des Verschlusses in Ausgangslage mit dem distalen Ende des Aufnahmeschlauches übereinstimmt oder gegenüber dem Ende des Aufnahmeschlauches zurücksteht.

Durch einen solchen den Katheter ergänzenden oder fortsetzenden und zu ihm gehörenden Aufnahmeschlauch ist eine besonders gute Anpassung an ein Verschlusselement möglich, welches erst nach dem Ausschieben aus dem Katheter seine endgültige Form erlangt, also innerhalb des Katheters zunächst in eine längliche Form gebracht und in dieser gehalten wird. Ein Aufnahmeschlauch erlaubt es, zusammen mit dem in ihm befindlichen, zunächst gegenüber seiner Gebrauchsstellung verformten Verschluss bei der Implantation gut einem Blutgefäß folgen zu können, also entsprechende Biegebewegungen durchzuführen.

Besonders günstig ist es dabei, wenn der Aufnahmeschlauch den Verschluss und dessen Befestigungsstelle an dem Schiebeelement in sich aufnimmt. Somit ist der gesamte Verschluss und seine Kupplungsstelle oder Stelle der lösbaren Befestigung an dem Schiebeelement in dem Aufnahmeschlauch untergebracht, der sich mit seiner Schlauchstruktur gut an die Außenseite des Verschlusselementes und seiner Befestigungsstelle anpassen kann.

Günstig ist es, wenn der Aufnahmeschlauch einen Querschnitt hat, der den Querschnitt des übrigen Katheters übertrifft. Entsprechend schlank und dünn kann der Katheter bemessen sein, während der Aufnahmeschlauch zur Aufnahme des Verschlusses eine etwas größere Querschnitts-Ausdehnung haben kann.

Das Einführen des Katheters und des von ihm transportierten Verschlusses bis in das Innere des Herzens kann dadurch erleichtert sein, dass die Vorrichtung einen Führungsdraht aufweist beziehungsweise zu der Vorrichtung ein Führungsdraht gehört, der vor dem Katheter zum Beispiel in ein Blutgefäß einführbar ist, und dass an dem Katheter und/oder an dem Aufnahmeschlauch zumindest nahe dem oder an dem distalen Ende eine Führung für diesen Führungsdraht vorgesehen ist. Mit Hilfe eines Führungsdrahtes kann der Katheter leichtgängig und bequem in das Herz eingeführt werden, ohne dass dabei aber Relativbewegungen zwischen dem Katheter und dem Verschluss notwendig sind, weil sich der Verschluss am distalen Ende des Katheters oder des Aufnahmeschlauches befindet.

Die Führung für den Führungsdraht kann eine etwa in radialer Richtung verlaufende oder schräge Öffnung sein, die quer zur Wandung des Aufnahmeschlauches orientiert ist oder diese durchsetzt. Dabei ist auch möglich, dass die Führung für den Führungsdraht durch eine eine entsprechende Öffnung aufweisende Hülse oder Öse gebildet ist. Hat der Aufnahmeschlauch selbst eine entsprechende Lochung als Öffnung für den Führungsdraht, ist die Anbringung einer entsprechenden Gegenführung entbehrlich. Eine angebrachte Öse oder Hülse verbessert hingegen die Führung.

Eine zweckmäßige Ausführungsform kann dabei vorsehen, dass eine als Führung dienende Öffnung durch die Wandung des Aufnahmeschlauches mit Abstand zu dessen Ende im Bereich des distalen Endes des vor oder bei dem Implantieren von dem Aufnahmeschlauch aufgenommenen Verschlusses angeordnet ist und der zwischen dieser Öffnung und dem distalen Ende des Schlauches verlaufende Teil des Führungsdrahtes in dem Abstand des distalen Endes des Verschlusselements zum distalen Ende des Aufnahmeschlauches in dessen Innerem verläuft. Es ist also möglich, das distale Ende des Verschlusses in Ausgangslage mit Abstand zum distalen Ende des Katheters beziehungsweise des Aufnahmeschlauches anzuordnen und den Führungsdraht in diesem Bereich des Katheters oder Aufnahmeschlauches in dessen Inneren anzuordnen, der dadurch von dem Verschluss frei bleibt. Der Führungsdraht kann also aus dem Katheter oder Aufnahmeschlauch nach außen an einer Stelle durchtreten, die von dem Verschluss weitestgehend frei ist, so dass die Verschiebung des Katheters und des Aufnahmeschlauches entlang dem Führungsdraht nicht zu einer zusätzlichen Reibung zwischen Führungsdraht und Verschluss führt.

Dabei kann es zweckmäßig sein, wenn eine hülsenartige Führung an der Außenseite zumindest des Aufnahmeschlauches verläuft und der Führungsdraht nach dem Austritt aus der Führungshülse außenseitig oder über eine Eintrittsöffnung im Inneren des Katheters weiter verläuft. Auch dadurch kann eine Reibung zwischen Verschluss und Führungsdraht während des Implantationsvorgangs vermieden werden.

Das Schiebeelement kann mit seiner Befestigungsstelle für den Verschluss über das distale Ende des Katheters und/oder des Aufnahmeschlauches hinaus verschiebbar, dann der Verschluss mit dem Verschiebeelement verbindbar, insbesondere mittels einer Gewindeverbindung verschraubbar und dann in die Implantierlage im Inneren des distalen Endes des Katheters oder seines Aufnahmeschlauches einziehbar sein.

Da eine lösbar oder trennbare Verbindung zwischen Schiebeelement und Verschluss vorgesehen ist, kann diese Verbindung auch zunächst hergestellt werden, indem die Befestigungsstelle des Schiebeelementes durch eine entsprechend weite Verschiebung über das distale Ende des Katheters hinaus befördert wird, wo der Verschluss bequem angebracht werden kann. Durch Zurückziehen gelangt dann der Verschluss in seine während des Implantiervorganges vorgesehene Gebrauchslage. Dies kann bei einer Fertigung im Vorfeld der Benutzung der Vorrichtung oder unmittelbar vor der Benutzung der Vorrichtung durch einen Arzt geschehen. In letzterem Falle könnte also der Verschluss separat von dem Katheter geliefert und erst unmittelbar vor der Benutzung in eine entsprechende Gebrauchsstellung gebracht werden.

Der den Verschluss vor und während der Implantation aufnehmende Aufnahmeschlauch kann gegenüber dem sich anschließenden Katheter innenseitig flüssigkeitsdicht abgeschlossen sein und das Schiebeelement kann relativ zu dieser Dichtung flüssigkeitsdicht verschiebbar sein. Somit wird weitestgehend verhindert, dass während der Implantation Blut in den Katheter eindringt.

Darüber hinaus ist es möglich, dass in dem zur Aufnahme des Verschlusses dienenden Ende des Katheters oder in im Aufnahmeschlauch eine Füllung aus einer blutverträglichen Flüssigkeit, insbesondere eine Kochsalzlösung, eingefüllt ist. Dadurch kann das Eintragen von Luft während des Implantationsvorganges mit größerer Sicherheit vermieden werden.

Der Aufnahmeschlauch kann kleine Lochungen haben, durch die beim Einfüllen der die Luft verdrängenden Flüssigkeit oder Kochsalzlösung vor dem Implantieren die Luft aus diesem Aufnahmeschlauch verdrängbar ist, wobei die Lochungen so klein gewählt sind, dass die Luft durch sie verdrängbar ist, die Flüssigkeit aber aufgrund ihrer Oberflächenspannung oder der Größe ihrer Moleküle im Inneren des Aufnahmeschlauches verbleibt. Eine solche Anordnung verlangt das Einfüllen der Flüssigkeit oder Kochsalzlösung mit Druck, damit die Luft durch die relativ kleinen Löcher auch tatsächlich verdrängt wird. Entsprechend groß ist die Sicherheit, die Luft vollständig verdrängt zu haben.

Eine weitere Ausgestaltung der Vorrichtung kann vorsehen, dass der Aufnahmeschlauch eine Vorkrümmung hat und durch Drehen des Katheters um seine Längsachse steuerbar ist. Entsprechend gut kann der Katheter mit dem Aufnahmeschlauch zum Inneren des Herzens vorgeschoben werden, wobei aufgrund der Vorkrümmung des Aufnahmeschlauches auf einen Führungsdraht verzichtet werden kann, aber auch die Implantation mit Hilfe eines Führungsdrahtes unterstützt werden kann.

Eine weitere Ausgestaltung der Vorrichtung, insbesondere einer einen Führungsdraht aufweisenden Vorrichtung kann vorsehen, dass der Führungsdraht an seinem distalen Ende eine Erweiterung oder einen Anschlag hat, der gegenüber der Führungsöffnung oder einer Führungshülse oder -öse einen vergrößerten Querschnitt hat, und dass der Aufnahmeschlauch durch eine Zugkraft an dem Führungsdraht durch dessen exzentrische Anordnung zur Mitte des Aufnahmeschlauches biegbar ist. Somit kann der Führungsdraht eine Zusatzfunktion erhalten, weil er zur Steuerung des Katheters während der Implantation benutzt werden kann, beispielsweise dann, wenn der Katheter und der Aufnahmeschlauch schon über eine größere Strecke des Führungsdrahtes verschoben sind und dann eine Auslenkung des distalen Endes vorteilhaft erscheint.

Der Aufnahmeschlauch kann aus Kunststoff bestehen oder als dünnwandige Metallhülse oder als Wendel oder als Geflecht, gegebenenfalls mit einem Überzug, ausgebildet sein. Entsprechend stabil einerseits und flexibel andererseits ist dieser zu dem Katheter gehörende Aufnahmeschlauch.

Dabei kann der Rand des distalen Endes des Aufnahmeschlauches zur Erleichterung der Einführung abgerundet oder abgeschrägt sein oder das gesamte Ende des Aufnahmeschlauches kann - ähnlich wie bei einer Kanüle - abgeschrägt und/oder nachgiebig weich ausgebildet sein. Auch dies begünstigt das Einführen des Katheters und seines Aufnahmeschlauches mit dem darin befindlichen Verschluss.

Vor allem bei Kombination einzelner oder mehrerer der vorbeschriebenen Merkmale und Maßnahmen ergibt sich eine Vorrichtung zum Implantieren eines Verschlusses in eine ungewollte Öffnung im Inneren des Herzens, bei welcher für die Relativbewegung zwischen dem Verschluss und dem ihn aufnehmenden Katheter oder Aufnahmeschlauch nur eine sehr kurze Verschiebebewegung erforderlich ist, also die Gefahr von Abrieb aus dem Inneren des Katheters durch das Verschieben des Verschlusses in dessen Gebrauchsstellung weitestgehend vermieden wird.

Nachstehend sind Ausführungsbeispiele der Erfindung anhand der Zeichnung näher beschrieben. Es zeigt in zum Teil schematisierter Darstellung:
- Fig. 1: eine erfindungsgemäße Vorrichtung zum Implantieren eines Verschlusses in eine ungewollte Öffnung in der Trennwand zwischen den beiden Vorhöfen eines Herzens, wobei ein zu der Vorrichtung gehörender Katheter durch ein Blutgefäß bis in das Herz eingeführt ist und der Verschluss bereits mit Hilfe eines im Inneren des Katheters angeordneten Schiebeelements in seine Gebrauchsstellung ausgeschoben aber noch nicht von dem Schiebeelement getrennt ist,
- Fig. 2: eine Seitenansicht der erfindungsgemäßen Vorrichtung, wobei das Schiebeelement mit der Befestigungsstelle für den Verschluss über das distale Ende des Katheters und eines dort befindlichen Aufnahmeschlauches hinaus verschoben ist, bevor der Verschluss daran befestigt wird,
- Fig. 3: in vergrößertem Maßstab einen Längsschnitt des distalen Endes des Katheters und des Aufnahmeschlauches mit dem vorgeschobenen Schiebeelement gemäß Fig. 2,
- Fig. 4: eine der Fig. 2 entsprechende Darstellung nach dem Befestigen und Aufschrauben des Verschlusses auf das distale Ende des Schiebeelements und ein dort vorgesehenes Gewinde,
- Fig. 5: eine der Fig. 4 entsprechende Darstellung, nachdem das Schiebeelement und der Verschluss teilweise zurückgezogen sind und noch ein Teil des Verschlusses über das distale Ende des Katheters oder des Aufnahmeschlauches vorsteht,
- Fig. 6: eine den Fig. 2, 4 und 5 entsprechende Darstellung, bei welcher der Verschluss und das Schiebeelement in Gebrauchsstellung, das heißt in der Lage angeordnet sind, in welcher die Implantation durchführbar ist.
- Fig. 7: eine der Fig. 3 entsprechende Darstellung, also einen Längsschnitt des distalen Endes des Katheters und des Aufnahmeschlauches mit dem darin angeordneten, durch den Aufnahmeschlauch verformten Verschlusselement, welches aus dieser Lage gemäß Fig. 1 in Richtung von einem Handgriff der Vorrichtung weg in die Öffnung des Herzens einschiebbar ist, um beispielsweise mit zwei miteinander verbundene Schirme eine Anlage an den Rändern der Öffnung im Inneren des Herzens an beiden Seiten der Trennwand zu ermöglichen,
- Fig. 8: eine abgewandelte Ausführungsform in der Darstellung gemäß Fig. 6, wobei ein Führungsdraht vorgesehen ist, der weitgehend entlang der Außenseite des Katheters verläuft und mit einer Führungsöffnung nahe dem distalen Ende des Aufnahmeschlauchs zusammenwirkt,
- Fig. 9: in vergrößertem Maßstab einen Längsschnitt des distalen Endes des Katheters und des Aufnahmeschlauches mit dem darin befindlichen verformten Verschluss und der Anordnung des Führungsdrahtes, der im Bereich des distalen Endes des eingezogenen Verschlusses durch die Wandung des Aufnahmeschlauches in dessen Inneres eintritt,
- Fig. 10: eine der Fig. 9 entsprechende Darstellung einer abgewandelten Ausführungsform, bei welcher der Führungsdraht durch eine hülsenartige Führung an der Außenseite des Aufnahmeschlauches verläuft,
- Fig. 11: eine Seitenansicht des distalen Endes des Katheters und des Aufnahmeschlauches mit einer abgewandelten Anordnung des Führungsdrahtes, der am Übergang zwischen Aufnahmeschlauch und Katheter in das Innere des Katheters übergeht,
- Fig. 12: eine Seitenansicht des distalen Endes des Katheters und des Aufnahmeschlauches analog Fig. 10, wobei am distalen Ende des Führungsdrahtes ein Anschlag angeordnet ist, der mit dem distalen Ende der hülsenförmigen Führung zusammenwirken kann, sowie
- Fig. 13: eine der Fig. 12 entsprechende Darstellung nach dem Zurückziehen des Führungsdrahtes, bis der Anschlag mit dem distalen Ende der hülsenartige Führung derart zusammenwirkt, dass eine Zugkraft auf den Führungsdraht zu einer Krümmung des distalen Endes des Katheters beziehungsweise des Aufnahmeschlauches führt.

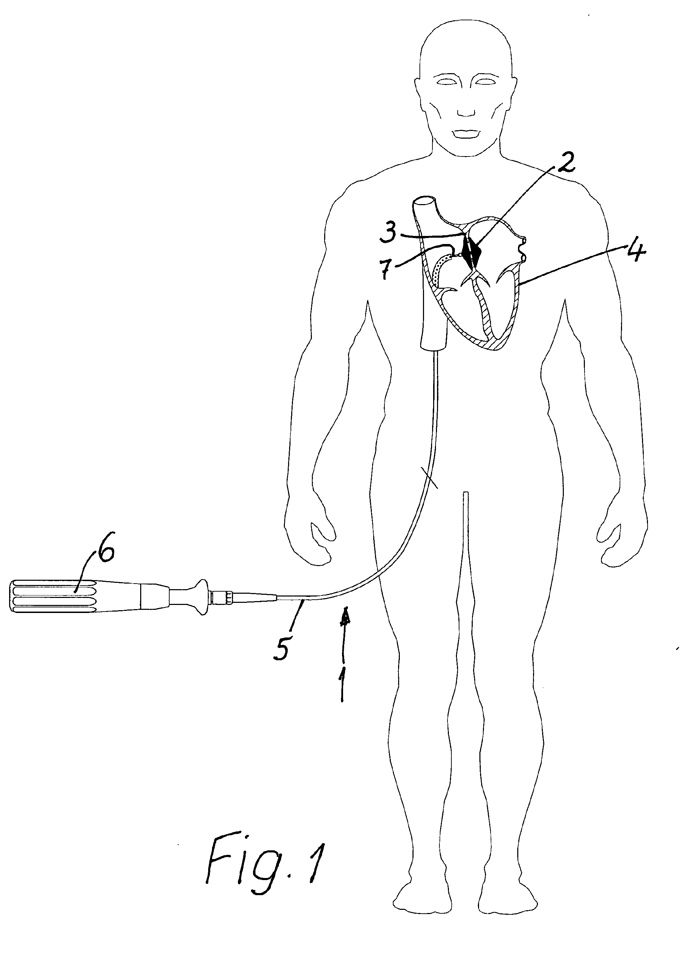

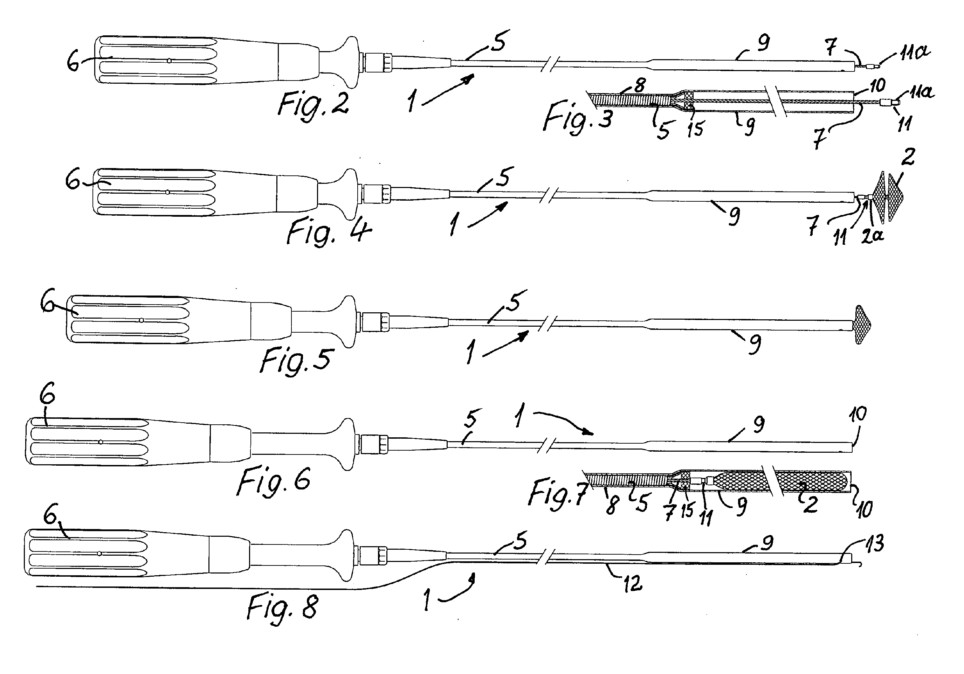

Eine im Ganzen mit 1 bezeichnete Vorrichtung dient zum Implantieren eines Verschlusses 2 in eine ungewollte Öffnung der Trennwand 3 zwischen den beiden Vorhöfen eines Herzens 4. Solche Verschlüsse 2 sind bekannt und haben gemäß Fig. 1 und 4 in Gebrauchsstellung die Form von zwei etwas beabstandeten, gegensinnigen Schirmen, die die Herztrennwand 3 und die ungewollte Öffnung zwischen sich aufnehmen.

Wesentlicher Bestandteil der Vorrichtung 1 ist ein im Ganzen mit 5 bezeichneter Katheter, der gemäß Fig. 1, 2, 4 bis 6 und 8 einen Handgriff 6 für seine Betätigung hat.

Ferner gehört zu der Vorrichtung 1 ein im Inneren des Katheters 5 in dessen Längserstreckungsrichtung bewegbares, vor allem in den Fig. 3 und 4 erkennbares Schiebeelement 7, das im Ausführungsbeispiel als Litze ausgebildet ist, also biegsam ist und die Biegungen des Katheters 5 bei der Implantation mitmachen kann. Am distalen Ende des Schiebeelements 7 ist gemäß den Fig. 2 bis 4 sowie 9 und 10 der Verschluss 2 lösbar anbringbar oder lösbar befestigt und kann durch die erwähnte Verschiebebewegung aus der in den Fig. 6 bis 13 vorgesehenen Ausgangslage in die in Fig. 1 dargestellte Gebrauchsstellung gebracht werden. Ist diese Lage gemäß Fig. 1 erreicht, kann das Schiebeelement 7 von dem Verschluss 2 getrennt oder gelöst und wieder zurückgezogen werden.

Damit für die Verschiebung des Verschlusses 2 kein zu großer Schiebeweg benötigt wird und die Gefahr, dass der metallische, aus einem Geflecht gebildete Verschluss 2 bei einer solchen Verschiebung Partikel von der Innenseite des Katheters 5 ablöst oder abreibt, vermieden wird, ist der mit dem Schiebeelement 7 verbundene Verschluss 2 vor und für den Implantationsvorgang gemäß den Fig. 6 bis 11 im distalen Endbereich des Katheters 5 in dessen Innerem angeordnet. Entsprechend kurz ist die zum Einführen in die Öffnung der Trennwand 3 im Herzen 4 erforderliche Verschiebebewegung für den Verschluss 2 während des Implantationsvorganges.

In den Fig. 3, 7, 9 und 10 ist angedeutet, dass der Katheter 5 eine flexible Wendel enthält oder durch eine solche Wendel gebildet ist, die noch mit einem Schlauch 8 überzogen sein kann, so dass der Katheter 5 die gewünschte hohe Biegbarkeit hat, um Richtungsänderungen der Gefäße folgen zu können, durch welche hindurch die Implantation erfolgt.

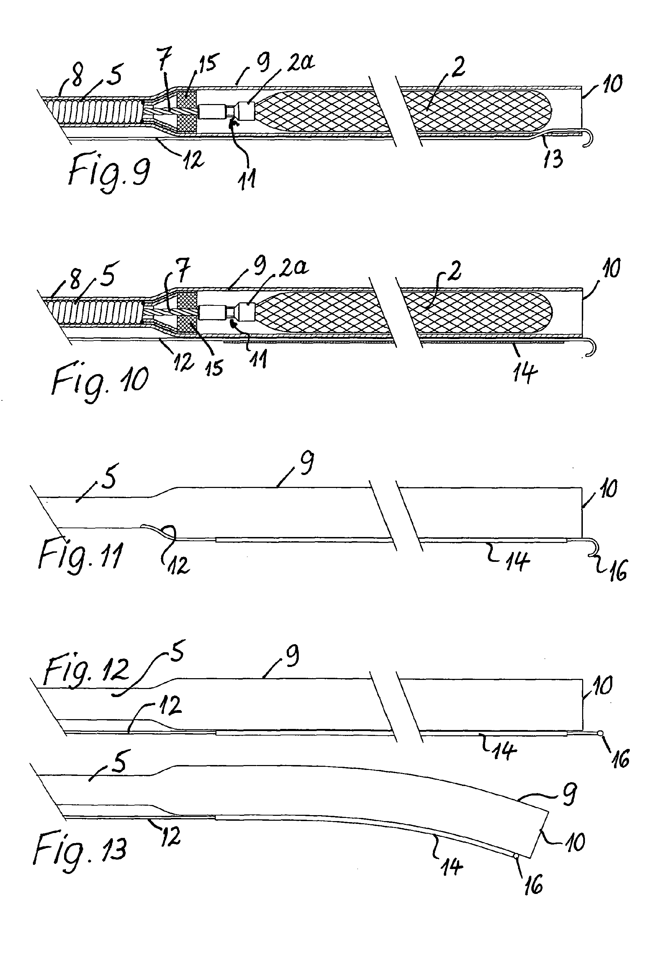

Das distale Ende des Katheters 5 ist in allen Ausführungsbeispielen durch einen dort angeordneten Aufnahmeschlauch 9 gebildet, der gemäß den Fig. 8, 9 und 10 die Fortsetzung des Schlauches 8 sein kann. In jedem Fall ist dieser das distale Ende des Katheters 5 bildende Aufnahmeschlauch 9 mit dem übrigen Katheter 5 verbunden und nimmt den Verschluss 2 in Ausgangslage derart in sich auf, dass das distale Ende des Verschlusses 2 in dieser Ausgangslage mit dem distalen Ende 10 des Aufnahmeschlauches 9 übereinstimmt oder gemäß Fig. 9 und 10 bevorzugt diesem Ende 10 gegenüber zurücksteht. Man erkennt in den Fig. 9 und 10 einen geringfügigen Abstand zwischen dem distalen Ende des in dem Aufnahmeschlauch 9 befindlichen Verschlusses 2 von dem distalen Ende 10 des Aufnahmeschlauches 9. Somit ist sichergestellt, dass der Verschluss 2 bei der Implantation des Katheters 5 und damit auch des Verschlusses 2 gut geschützt im Inneren des Aufnahmeschlauches 9 verbleibt und über seine ganze Länge umschlossen ist.

Dabei nimmt der Aufnahmeschlauch 9 den Verschluss 2 und dessen Befestigungsstelle 11 an dem Schiebeelement 7 in sich auf, das heißt diejenige Stelle 11, an welcher der Verschluss 2 mit dem Schiebeelement 7 lösbar verbunden und befestigt ist, befindet sich ebenfalls im Inneren des Aufnahmeschlauches 9.

Dabei wird anhand der Zeichnung und insbesondere der Fig. 9 und 10 auch deutlich, dass der Aufnahmeschlauch 9 einen Querschnitt hat, der den Querschnitt des übrigen Katheters 5 übertrifft, so dass er einen entsprechend voluminösen Verschluss 2 gut umschließen und in sich aufnehmen kann. Die Befestigungsstelle 11 ist auch in Fig. 3 erkennbar, wobei dort der Verschluss 2 noch nicht damit verbunden ist und es kann dort beispielsweise ein stiftförmiges Teil 11a mit einem Außengewinde vorgesehen sein, das in ein Innengewinde an einer Hülse 2a passt, die zu dem Verschluss 2 gehört und dessen Geflecht zusammenhält.

In den Ausführungsbeispielen gemäß Fig. 8 bis 13 ist dargestellt, dass die Vorrichtung 1 einen Führungsdraht 12 aufweisen kann, also ein Führungsdraht 12 zu der Vorrichtung 1 gehört, der in üblicher Weise vor dem Katheter 5 in ein entsprechendes Blutgefäß und in das Herz 4 einführbar ist. An dem Katheter 5 oder bevorzugt an dem Aufnahmeschlauch 9 ist zumindest nahe dem oder an dem distalen Ende 10 eine Führung für diesen Führungsdraht 12 vorgesehen, die unterschiedlich ausgebildet sein kann und es ermöglicht, dass der Katheter 5 bei seiner Implantation dem zuvor implantierten Führungsdraht bis in das Herz 4 folgt.

Im Ausführungsbeispiel gemäß den Fig. 8 und 9 ist die Führung für den Führungsdraht 12 eine etwa in radialer Richtung verlaufende oder schräge Öffnung 13, die quer zur Wandung des Aufnahmeschlauches 9 orientiert ist und diese durchsetzt. Dies wird vor allem in Fig. 9 durch eine zweimalige Richtungsänderung des Führungsdrahtes 12 deutlich, der zunächst an der Außenseite des Katheters 5 und des Aufnahmeschlauches 9 verläuft und dann durch die Öffnung 13 in das Innere des Aufnahmeschlauches 9 übergeht, an der Innenseite entlang verläuft und dann das Ende 10 übergreift.

In den Ausführungsbeispielen gemäß Fig. 10 bis 13 ist die Führung für den Führungsdraht 12 durch eine eine entsprechende Öffnung aufweisende biegsame Hülse 14 oder Öse gebildet. Dies ergibt eine längere gegenseitige Führung, erfordert aber zusätzlich diese Hülse 14.

Gemäß Fig. 9 ist die als Führung dienende Öffnung 13 durch die Wandung des Aufnahmeschlauches 9 mit Abstand zu dessen distalem Ende 10 im Bereich des distalen Endes des vor oder bei dem Implantieren von dem Aufnahmeschlauch 9 aufgenommenen und umschlossenen Verschlusses 2 angeordnet und der zwischen dieser Öffnung 13 und dem distalen Ende 10 des Aufnahmeschlauches 9 verlaufende Teil des Führungsdrahtes 12 verläuft im Bereich dieses Abstandes des distalen Endes des Verschlusselements 2 zum distalen Ende 10 im Inneren des Aufnahmeschlauches 9. Dadurch kann eine Reibung zwischen Verschluss 2 und Führungsdraht 12 während des Implantierens des Katheters 5 vermieden werden.

In den Fig. 10 und 12 ist dargestellt, dass die hülsenartige Führung oder Führungshülse 14 an der Außenseite des Aufnahmeschlauches 9 verläuft und der Führungsdraht 12 nach dem Austritt aus dieser Führungshülse 14 - vom distalen Ende des Aufnahmeschlauches 9 gesehen - außenseitig an dem Katheter 5 entlang weiterverläuft.

Fig. 11 zeigt eine vergleichbare Anordnung, bei welcher aber der Führungsdraht 12 nach dem Austritt aus der Führungshülse 14 durch eine Eintrittsöffnung in das Innere des Katheters 5 überführt ist und dort weiter zum proximalen Ende hin verläuft.

In den Fig. 2 bis 7 ist dargestellt, dass das Schiebeelement 7 mit seiner Befestigungsstelle 11 über das distale Ende 10 des Katheters 5 beziehungsweise des Aufnahmeschlauches 9 hinaus verschiebbar ist oder verschoben sein kann, so dass der Verschluss 2 bequem mit dem Schiebeelement 7 und der Befestigungsstelle 11 verbindbar, beispielsweise, wie bereits erwähnt, mittels einer Gewindeverbindung verschraubbar ist. Diese Anordnung zeigt Fig. 4. Gemäß Fig. 5 und 6 ist dann der Verschluss 2 in die Implantierlage im Inneren des distalen Endes des Katheters 5 oder des Aufnahmeschlauches 9 durch eine entsprechende Rückzugsbewegung an dem Schiebeelement 7 oder eine Vorschubbewegung des Katheters 5 relativ zu dem Schiebeelement 7 durchführbar, um die Ausgangslage gemäß Fig. 6 und 7 für den Implantationsvorgang herzustellen. Analoges gilt für die Ausführungsbeispiele gemäß Fig. 8 bis 13.

Dabei kann diese Verbindung des Verschlusses 2 mit dem Schiebeelement 7 schon bei der Fertigung der Vorrichtung 1 oder aber erst kurz vor dem Implantationsvorgang erfolgen.

In den Fig. 3, 7, 9 und 10 ist dargestellt, dass der den Verschluss 2 vor und während der Implantation aufnehmende Aufnahmeschlauch 9 gegenüber dem sich zum proximalen Ende hin anschließenden Katheter 5 innenseitig flüssigkeitsdicht abgeschlossen ist und das Schiebeelement oder der pusher 7 relativ zu der dazu dienenden Dichtung 15 flüssigkeitsdicht verschiebbar ist. Man erkennt in den genannten Figuren deutlich, wie das als Litze ausgebildete Schiebeelement 7 die Dichtung 15 durchsetzt, welche dabei dichtend an dem Schiebeelement 7 anliegt.

Dadurch ist es möglich, dass in dem zur Aufnahme des Verschlusses 2 dienenden Ende des Katheters 5, in den Ausführungsbeispielen in dem Aufnahmeschlauch 9 eine Füllung aus einer blutverträglichen Flüssigkeit, zum Beispiel aus einer Kochsalzlösung eingefüllt ist, um Luft zu verdrängen. Dabei kann der Aufnahmeschlauch 9 in in der Zeichnung nicht erkennbarer Weise kleine Lochungen haben, durch die beim Einfüllen der die Luft verdrängenden Flüssigkeit oder Kochsalzlösung vor dem Implantieren die Luft aus diesem Aufnahmeschlauch 9 verdrängbar ist, weil das Einfüllen unter Druck erfolgt, wobei die Lochungen so klein gewählt sind, dass die Luft durch sie verdrängbar ist, die Flüssigkeit aber aufgrund ihrer Oberflächenspannung und/oder der Molekülgrößen im Inneren des Aufnahmeschlauches 9 verbleibt.

In Fig. 13 ist eine Krümmung des Aufnahmeschlauches 9 dargestellt, die entgegen der Darstellung der Fig. 13 eine Vorkrümmung sein könnte, um durch Drehen des Katheters 5 um seine Längsachse bei dem Implantiervorgang eine Steuerung zu ermöglichen. Eine solche Vorkrümmung würde diese Steuerung erlauben, auch wenn kein Führungsdraht 12 vorhanden ist.

In den Fig. 12 und 13 ist dargestellt, dass der Führungsdraht 12 an seinem distalen Ende eine Erweiterung oder einen Anschlag 16 haben kann, der gegenüber der Führungsöffnung 13 oder der Führungshülse 14 oder einer Führungshülse einen vergrößerten Querschnitt hat, also durch diese Öffnung 13 oder Hülse 14 oder Öse nicht hindurchpasst. Dadurch kann der Aufnahmeschlauch 9 durch eine Zugkraft an dem Führungsdraht 12 durch dessen exzentrische Anordnung zur Mitte des Aufnahmeschlauches 9 in der in Fig. 13 erkennbaren Weise gekrümmt oder gebogen werden, um dadurch eine Steuerung während der Implantation zu erlauben.

Der in den Ausführungsbeispielen dargestellte Aufnahmeschlauch 9 besteht zweckmäßigerweise aus Kunststoff, kann aber auch aus einem dünnwandigen, entsprechend geformten und verbundenen Metallblech bestehen, also als dünnwandige Metallhülse ausgebildet sein. Ferner kann der Aufnahmeschlauch 9 auch als Wendel oder als Geflecht gegebenenfalls mit einem Überzug ausgebildet sein, um eine entsprechend gute Festigkeit zu haben. Der Rand des distalen Endes 10 des Aufnahmeschlauches 9 kann bei den verschiedenen Ausführungsbeispielen zusätzlich abgerundet oder abgeschrägt sein, um das Einführen in Engstellen oder Abzweigungen von Blutgefäßen zu erleichtern.

Außerdem ist es möglich, dass das gesamte Ende 10 nicht, wie in den Figuren dargestellt, rechtwinklig zur Längsmittelachse, sondern schräg dazu angeordnet ist, also ähnlich wie bei einer Kanüle abgeschrägt ist. Dabei kann dieses Ende 10 und sein Rand zweckmäßigerweise nachgiebig weich ausgebildet sein, was ebenfalls den Implantiervorgang erleichtert.

Die Vorrichtung 1 dient zum Implantieren eines Verschlusses 2 in eine ungewollte Öffnung in einer Trennwand 3 in einem Herzen 4 und weist einen Katheter 5 mit einem in dessen Längsrichtung im Inneren bewegbaren Schiebeelement 7 (pusher) auf, an dessen distalem Ende der Verschluss 2 lösbar befestigt ist und in seine Gebrauchsstellung gebracht werden kann, wo das Schiebeelement 7 von dem Verschluss 2 wieder gelöst wird. Dabei ist vorgesehen, dass der mit dem Schiebeelement 7 verbundene Verschluss 2 für den Implantationsvorgang im distalen Endbereich des Katheters 5 angeordnet und von diesem Endbereich ganz oder wenigstens teilweise umschlossen ist.

## Patentansprüche

1. Vorrichtung (1) zum Implantieren eines Verschlusses (2) in einer ungewollten Öffnung der Trennwand (3) zwischen den beiden Vorhöfen eines Herzens (4) mit einem Katheter (5) und mit einem in diesem Katheter in dessen Längserstreckungsrichtung bewegbaren Schiebeelement (7), an dessen distalem Ende der Verschluss (2) lösbar anbringbar oder lösbar befestigt und durch die Verschiebebewegung aus dem distalen Ende des Katheters (5) in seine Gebrauchsstellung bringbar und dann von dem Schiebeelement (7) trennbar ist, **dadurch gekennzeichnet, dass** der mit dem Schiebeelement (7) verbundene Verschluss (2) für, vor und bei dem Implantationsvorgang im distalen Endbereich des Katheters (5) in dessen Innerem angeordnet und von diesem Endbereich ganz oder wenigstens teilweise umschlossen ist.

2. Vorrichtung nach Anspruch 1, **dadurch gekennzeichnet, dass** der Katheter (5) eine flexible Wendel enthält oder durch eine solche Wendel gebildet ist.

3. Vorrichtung nach Anspruch 1 oder 2, **dadurch gekennzeichnet, dass** das distale Ende des Katheters (5) durch einen dort angeordneten Aufnahmeschlauch (9) gebildet ist, der mit dem übrigen Katheter (5) verbunden ist und den Verschluss (2) zumindest teilweise derart in sich aufnimmt, dass das distale Ende des Verschlusses (2) in Ausgangslage mit dem distalen Ende (10) des Aufnahmeschlauches (9) übereinstimmt oder diesem gegenüber zurücksteht.

4. Vorrichtung nach einem der Ansprüche 1 bis 3, **dadurch gekennzeichnet, dass** der Aufnahmeschlauch (9) den Verschluss (2) und dessen Befestigungsstelle (11) an dem Schiebeelement (7) in sich aufnimmt.

5. Vorrichtung nach einem der Ansprüche 1 bis 4, **dadurch gekennzeichnet, dass** der Aufnahmeschlauch (9) einen Querschnitt hat, der den Querschnitt des übrigen Katheters (5) übertrifft.

6. Vorrichtung nach einem der Ansprüche 1 bis 5, **dadurch gekennzeichnet, dass** sie einen Führungsdraht (12) aufweist, der vor dem Katheter (5) zum Beispiel in ein Blutgefäß einführbar ist, und dass an dem Katheter (5) und/oder an dem Aufnahmeschlauch (9) zumindest nahe dem oder an dem distalen Ende (10) eine Führung für diesen Führungsdraht (12) vorgesehen ist.

7. Vorrichtung nach einem der Ansprüche 1 bis 6, **dadurch gekennzeichnet, dass** die Führung eine etwa in radialer Richtung verlaufende oder schräge Öffnung (13) ist, die quer zur Wandung des Aufnahmeschlauches (9) orientiert ist oder diese durchsetzt.

8. Vorrichtung nach einem der Ansprüche 1 bis 7, **dadurch gekennzeichnet, dass** die Führung für den Führungsdraht (12) durch eine eine entsprechende Öffnung aufweisende Hülse (14) oder Öse gebildet ist.

9. Vorrichtung nach einem der Ansprüche 1 bis 8, **dadurch gekennzeichnet, dass** eine als Führung dienende Öffnung (13) durch die Wandung des Aufnahmeschlauches (9) mit Abstand zu dessen Ende (10) im Bereich des distalen Endes des vor oder bei dem Implantieren von dem Aufnahmeschlauch (9) aufgenommenen Verschlusses (2) angeordnet ist und der zwischen dieser Öffnung (13) und dem distalen Ende (10) des Aufnahmeschlauches (9) verlaufende Teil des Führungsdrahtes (12) in dem Abstand des distalen Endes des Verschlusselements (2) zum distalen Ende (10) des Aufnahmeschlauches in dessen Innerem verläuft.

10. Vorrichtung nach einem der Ansprüche 1 bis 9, **dadurch gekennzeichnet, dass** die hülsenartige Führung (14) an der Außenseite zumindest des Aufnahmeschlauches (9) verläuft und der Führungsdraht (12) nach dem Austritt aus der Führungshülse (14) außenseitig oder über eine Eintrittsöffnung im Inneren des Katheters (5) weiter verläuft.

11. Vorrichtung nach einem der Ansprüche 1 bis 10, **dadurch gekennzeichnet, dass** das Schiebeelement (7) mit seiner Befestigungsstelle (11) über das distale Ende (10) des Katheters (5) und/oder des Aufnahmeschlauches (9) hinaus verschiebbar, dann der Verschluss (2) mit dem Schiebeelement (7) verbindbar, insbesondere mittels einer Gewindeverbindung verschraubbar und dann in die Implantierlage im Inneren des distalen Endes des Katheters (5) oder seines Aufnahmeschlauches (9) einziehbar ist.

12. Vorrichtung nach einem der Ansprüche 1 bis 11, **dadurch gekennzeichnet, dass** der den Verschluss (2) vor und während der Implantation aufnehmende Aufnahmeschlauch (9) gegenüber dem sich anschließenden Katheter (5) innenseitig flüssigkeitsdicht abgeschlossen ist und das Schiebeelement (7) relativ zu dieser Dichtung (15) flüssigkeitsdicht verschiebbar ist.

13. Vorrichtung nach einem der Ansprüche 1 bis 12, **dadurch gekennzeichnet, dass** in dem zur Aufnahme des Verschlusses (2) dienenden Ende des Katheters (5) oder in dem Aufnahmeschlauch (9) eine Füllung aus einer blutverträglichen Flüssigkeit, insbesondere eine Kochsalzlösung, eingefüllt ist.

14. Vorrichtung nach einem der Ansprüche 1 bis 13, **dadurch gekennzeichnet, dass** der Aufnahmeschlauch (9) kleine Lochungen hat, durch die beim Einfüllen der die Luft verdrängenden Flüssigkeit oder Kochsalzlösung vor dem Implantieren die Luft aus diesem Aufnahmeschlauch (9) verdrängbar ist, wobei die Lochungen so klein gewählt sind, dass die Luft durch sie verdrängbar ist, die Flüssigkeit aber aufgrund ihrer Oberflächenspannung im Inneren des Aufnahmeschlauches (9) verbleibt.

15. Vorrichtung nach einem der Ansprüche 1 bis 14, **dadurch gekennzeichnet, dass** der Aufnahmeschlauch (9) eine Vorkrümmung hat und durch Drehen des Katheters (5) um seine Längsachse steuerbar ist.

16. Vorrichtung nach einem der Ansprüche 1 bis 15, **dadurch gekennzeichnet, dass** der Führungsdraht (12) an seinem distalen Ende eine Erweiterung oder einen Anschlag (16) hat, der gegenüber der Führungsöffnung (13) oder einer Führungshülse (14) oder -öse einen vergrößerten Querschnitt hat, und dass der Aufnahmeschlauch (9) durch eine Zugkraft an dem Führungsdraht (12) durch dessen exzentrische Anordnung zur Mitte des Aufnahmeschlauches (9) biegbar ist.

17. Vorrichtung nach einem der Ansprüche 1 bis 16, **dadurch gekennzeichnet, dass** der Aufnahmeschlauch (9) aus Kunststoff besteht oder als dünnwandige Metallhülse oder als Wendel oder als Geflecht, gegebenenfalls mit einem Überzug, ausgebildet ist.

18. Vorrichtung nach einem der Ansprüche 1 bis 17, **dadurch gekennzeichnet, dass** der Rand des distalen Endes (10) des Aufnahmeschlauches (9) abgerundet oder abgeschrägt oder das gesamte Ende (10) abgeschrägt und/oder nachgiebig weich ausgebildet ist.
